# EUROPEAN PATENT SPECIFICATION

(11) **EP 3 943 092 B1**
(45) Date of publication and mention of the grant of the patent: **13.11.2024**
(21) Application number: 21193195.1
(22) Date of filing: 24.02.2015
(51) Int. Cl.: A61K 35/35, A61K 38/18, A61K 38/19, A61K 38/20, A61K 38/22, A61K 31/05, C12N 5/077, A61P 3/00, A61P 3/04, A61P 3/06, A61P 3/10, A61P 5/00, A61P 5/50, A61P 43/00, A61K 31/195, A61K 31/165, A61K 31/352, A61K 31/426, A61K 31/4422, A61K 31/475, A61K 31/4965, A61K 31/506, A61K 31/5415, A61K 31/603, A61P 25/00, A61P 17/00, A61P 19/06, A61P 15/00, A61P 9/12, A61P 1/16

(54) **COMPOSITIONS FOR USE IN INDUCING DIFFERENTIATION OF HUMAN BROWN ADIPOCYTE PROGENITORS**
ZUSAMMENSETZUNGEN ZUR VERWENDUNG BEI DER INDUKTION DER DIFFERENZIERUNG HUMANER BRAUNER ADIPOZYTEN
COMPOSITIONS DESTINÉES À INDUIRE LA DIFFÉRENTIATION DE PROGÉNITEURS DES ADIPOCYTES BRUNS HUMAINS

(30) Priority: 24.02.2014 US 201461966496 P
(43) Date of publication of application: 26.01.2022
(62) Divisional of application: 15752698.9
(73) Proprietor: Energesis Pharmaceuticals Inc., Cambridge, MA 02139 (US)
(72) Inventor: BOSS, Olivier D., BOSTON, 02199 (US); GULLICKSEN, P. Scott, MEDFORD, 02155 (US)
(74) Representative: Cabinet Becker et Associés

(56) References cited:
- WO-A1-2013/071050
- WO-A2-2011/054001
- WO-A2-2012/099999
- WO-A2-2012/156968
- US-A1- 2005 009 739
- US-A1- 2011 130 385
- US-A1- 2013 303 573
- US-B1- 6 242 563
- SAITO MASAYUKI: "Brown adipose tissue as a therapeutic target for human obesity", OBESITY RESEARCH & CLINICAL PRACTICE, vol. 7, no. 6, 1 December 2013 (2013-12-01), AMSTERDAM, NL, pages e432 - e438, XP055862280, ISSN: 1871-403X, DOI: 10.1016/j.orcp.2013.09.001
- STOA-BIRKETVEDT G. L ET AL: "H 2 -receptor antagonist reduces food intake and weight gain in rats by non-gastric acid secretory mechanisms", ACTA PHYSIOL SCAND, 1 January 1997 (1997-01-01), pages 489 - 494, XP055862430, Retrieved from the Internet <URL:https://onlinelibrary.wiley.com/doi/epdf/10.1046/j.1365-201X.1997.00249.x> [retrieved on 20211117]
- KIM JIYOONG ET AL: "Impact of Blockade of Histamine H2Receptors on Chronic Heart Failure Revealed by Retrospective and Prospective Randomized Studies", JOURNAL OF THE AMERICAN COLLEGE OF CARDIOLOGY, vol. 48, no. 7, 3 October 2006 (2006-10-03), pages 1378 - 1384, XP029653836, ISSN: 0735-1097, DOI: 10.1016/J.JACC.2006.05.069
- G STÙA-BIRKETVEDT: "Cimetidine reduces weight and improves metabolic control in overweight patients with Type 2 diabetes", 1 November 1998 (1998-11-01), XP055059713, Retrieved from the Internet <URL:http://www.nature.com/ijo/journal/v22/n11/pdf/0800721a.pdf> [retrieved on 20130416]
- OH JEE-EUN ET AL: "Inhibition of mouse brown adipocyte differentiation by second-generation antipsychotics", EXPERIMENTAL AND MOLECULAR MEDICINE, vol. 44, no. 9, 1 January 2012 (2012-01-01), KR, pages 545 - 553, XP055862709, ISSN: 1226-3613, Retrieved from the Internet <URL:https://www.nature.com/articles/emm201262.pdf> DOI: 10.3858/emm.2012.44.9.062
- MASAKI T ET AL: "The hypothalamic H"1 receptor: a novel therapeutic target for disrupting diurnal feeding rhythm and obesity", TRENDS IN PHARMACOLOGICAL SCIENCES, ELSEVIER, HAYWARTH, GB, vol. 27, no. 5, 1 May 2006 (2006-05-01), pages 279 - 284, XP027891650, ISSN: 0165-6147, [retrieved on 20060501]
- MESTRE TIAGO A. ET AL: "Famotidine, a Histamine H 2 Receptor Antagonist, Does Not Reduce Levodopa-Induced Dyskinesia in Parkinson's Disease: A Proof-of-Concept Study", MOVEMENT DISORDERS CLINICAL PRACTICE, vol. 1, no. 3, 1 September 2014 (2014-09-01), pages 219 - 224, XP055862638, ISSN: 2330-1619, Retrieved from the Internet <URL:https://www.ncbi.nlm.nih.gov/pmc/articles/PMC6182979/pdf/MDC3-1-219.pdf> DOI: 10.1002/mdc3.12061
- REGLODI D ET AL: "Review on the Protective Effects of PACAP in Models of Neurodegenerative Diseases In Vitro and In Vivo", CURRENT PHARMACEUTICAL DESIGN, BENTHAM SCIENCE PUBLISHERS, NL, vol. 17, no. 10, 1 April 2011 (2011-04-01), pages 962 - 972, XP009195091, ISSN: 1381-6128, DOI: 10.2174/138161211795589355
- GRAY SARAH L. ET AL: "Temperature-Sensitive Phenotype in Mice Lacking Pituitary Adenylate Cyclase-Activating Polypeptide", ENDOCRINOLOGY, vol. 143, no. 10, 1 October 2002 (2002-10-01), US, pages 3946 - 3954, XP055902142, ISSN: 0013-7227, DOI: 10.1210/en.2002-220401

## Description

### TECHNICAL FIELD

The present disclosure relates to compositions and methods related to enhancing brown adipocytes, and/or brown adipocyte mass. Specifically, the present disclosure identifies and describes compounds that increase or promote the differentiation of brown adipose tissue (BAT) progenitor cells isolated from skeletal muscle into brown adipocytes. Further, the present disclosure identifies and describes compounds which interact with gene products involved in the regulation of brown adipocyte differentiation and/or mass. The invention is as defined in the claims.

### BACKGROUND

The epidemic of obesity is closely associated with increases in the prevalence of diabetes, hypertension, coronary heart disease, cancer and other disorders. The role of white adipose tissue is to store lipids, and it is associated with obesity. The role of brown adipose tissue ("BAT") is effectively the opposite. It is specialized in lipid combustion and the dissipation of energy as heat. Indeed, the brown adipocyte contains numerous mitochondria (in which cellular combustion occurs) and uniquely expresses uncoupling protein-1 ("UCP1"). UCP1 acts as an uncoupler of oxidative phosphorylation, resulting in dissipation of energy as heat. The sympathetic nervous system stimulates mitochondriogenesis and UCP 1 expression and activity. BAT-associated thermogenesis in rodents is increased upon exposure to low temperature (e.g., preventing hypothermia) or as a result of overeating, burning excess absorbed fat and preventing weight gain. BAT, by modifying susceptibility to weight gain and by consuming large amounts of glucose, also improves insulin sensitivity. It therefore plays an important role in the maintenance of body temperature, energy balance and glucose metabolism.

Experiments with transgenic animals support the potential anti-obesity properties of BAT. For example, the genetic ablation of BAT has been reported to cause obesity, while genetic increase in the amount and/or function of BAT (and/or UCP 1 expression) reportedly promotes a lean and healthy phenotype. Specifically, mice with a higher amount of BAT gain less weight and are more insulin-sensitive than control mice. Recently, ectopic BAT depots were evidenced in the mouse muscle, which have been shown to provide a genetic mechanism of protection from weight gain and metabolic syndrome.

Although UCP1 is reported to play a role in the control of energy balance in rodents and UCP1-expressing BAT is present in human neonates, it has long been thought that there was no physiologically relevant UCP1 expression in adult humans. Indeed, UCP 1-expressing BAT was thought to disappear early in life, and adult humans were thought to be devoid of BAT. Recently however, numerous studies have demonstrated that BAT is indeed maintained in most adult humans, albeit at considerably lower levels than in neonates and children.

As such, a need exists to carefully identify and study ways to provide more BAT in the adult body and/or stimulate UCP1 expression, for the study, prevention and treatment of various metabolic diseases such as obesity, type 2 diabetes, insulin-resistance, dyslipidemia and type 1 diabetes.

Applicants previously identified the presence of cells in various tissues that are capable of differentiating into brown adipocytes in e.g., PCT Publication No. WO2009151541 and WO2013071063. More particularly, WO2013071063 discloses an agent for use in promoting the differentiation of BAT progenitor cells obtained from skeletal muscle to brown adipocytes and the agent could be among others a PCAP related peptide. However, a need exists for agents (e.g., compounds, proteins, biologicals, and the like) that can, for example, induce the expression of the UCP1 gene, promote the differentiation of BAT progenitor cells into brown adipocytes in vitro, promote the differentiation of BAT progenitor cells to brown adipocytes in vivo, or combinations of these activities.

### SUMMARY

The references to methods of treatment in this description are to be interpreted as references to the compounds, pharmaceutical compositions and medicaments of the present invention for use in a method for treatment of the human (or animal) body by therapy.

The present invention relates to an ex vivo method of promoting brown adipogenesis using an agent being Pituitary adenylate cyclase-activating polypeptide (PACAP) propeptide of 55 aa (aa 25-79). Said method may comprise contacting a cell with the agent. Optionally, the cell is a brown adipose tissue (BAT) progenitor cell isolated from human skeletal muscle. Optionally, this cell is positive for CD34, and/or is negative for CD31.

### BRIEF DESCRIPTION OF THE DRAWINGS

FIG. 1 shows effects of various agents (incubated with brown adipocyte progenitor cells at day -3 to d0 and d0 to d3) on the expression of PPARy2 mRNA.
FIG. 2 shows effects of various agents (incubated with brown adipocyte progenitor cells at day -3 to d0 and d0 to d3) on the expression of PPARy2 mRNA.
FIG. 3 shows effects of various agents (incubated with brown adipocyte progenitor cells at day -3 to d0 and d0 to d3) on the expression of UCP1 mRNA.
FIG. 4 shows effects of various agents (incubated with brown adipocyte progenitor cells at day -3 to d0 and d0 to d3) on the expression of UCP1 mRNA.
FIG. 5 shows effects of various agents (incubated with brown adipocyte progenitor cells at day -3 to d0) on the expression of PPARy2 mRNA.
FIG. 6 shows effects of various agents (incubated with brown adipocyte progenitor cells at day -3 to d0) on the expression of PPARy2 mRNA.
FIG. 7 shows effects of various agents (incubated with brown adipocyte progenitor cells at day -3 to d0) on the expression of PPARy2 mRNA.
FIG. 8 shows effects of various agents (incubated with brown adipocyte progenitor cells at day -3 to d0 and d0 to d3) on the expression of PPARy2 mRNA.
FIG. 9 shows effects of various agents (incubated with brown adipocyte progenitor cells at day -3 to d0 and d0 to d3) on the expression of UCP1 mRNA.
FIG. 10 shows effects of FGF7 and FGF10 (both 100 nM, incubated with brown adipocyte progenitor cells at day -3 to d0 and d0 to d3) on the expression of UCP1 mRNA.
FIG. 11 shows effects of FGF7 and FGF10 (both 100 nM, incubated with brown adipocyte progenitor cells at day -3 to d0 and d0 to d3) on the expression of PPARy2 mRNA.
FIG. 12 shows effects of FGF7 (1 nM, incubated with brown adipocyte progenitor cells at day 0 to d3) on the expression of UCP1 mRNA. Rosiglitazone (rosi, 1 µM), bone morphogenic protein-7 (bmp7, 6 nM) or both rosi and bmp7were incubated with the cells at day -3 to d0.
FIG. 13 shows effects of FGF7 (1 nM, incubated with brown adipocyte progenitor cells at day 0 to d3) on the expression of PPARγ2 mRNA. Rosiglitazone (rosi, 1 µM), bone morphogenic protein-7 (bmp7, 6 nM) or both rosi and bmp7were incubated with the cells at day -3 to d0.
FIG. 14 shows effects of FGF10 (10 nM, incubated with brown adipocyte progenitor cells at day -3 to d0) on the expression of UCP1 mRNA. Rosiglitazone (rosi, 1 µM), bone morphogenic protein-7 (bmp7, 6 nM) or both rosi and bmp7were incubated with the cells at day -3 to d0.
FIG. 15 shows effects of FGF10 (10 nM, incubated with brown adipocyte progenitor cells at day -3 to d0) on the expression of PPARγ2 mRNA. Rosiglitazone (rosi, 1 µM), bone morphogenic protein-7 (bmp7, 6 nM) or both rosi and bmp7were incubated with the cells at day -3 to d0.
FIG. 16 shows effects of FGF7 (1 nM, incubated with brown adipocyte progenitor cells at day 0 to d3) or FGF10 (10 nM, incubated with the cells at day -3 to d0) on the expression of UCP1 mRNA. Rosiglitazone (rosi, 1 µM), bone morphogenic protein-7 (bmp7, 6 nM) or both rosi and bmp7 were incubated with the cells at day -3 to d0.
FIG. 17 shows effects of FGF7 (1 nM, incubated with brown adipocyte progenitor cells at day 0 to d3) or FGF10 (10 nM, incubated with the cells at day -3 to d0) on the expression of PPARγ2 mRNA. Rosiglitazone (rosi, 1 µM), bone morphogenic protein-7 (bmp7, 6 nM) or both rosi and bmp7were incubated with the cells at day -3 to d0.
FIG. 18 shows effects of various agents (incubated with brown adipocyte progenitor cells at day - 3 to d0 and d0 to d3) on the expression of PPARy2 mRNA.
FIG. 19 shows effects of various agents (incubated with brown adipocyte progenitor cells at day - 3 to d0 and d0 to d3) on the expression of UCP1 mRNA.

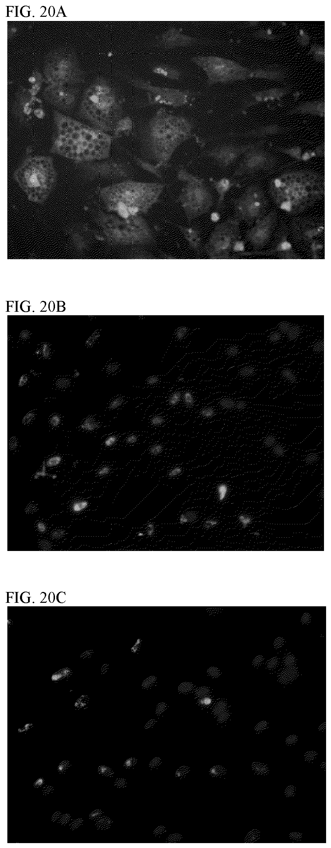

FIGS. 20A-20C show fluorescence microscopy pictures illustrating the immmunohistochemistry (IHC) assay results for UCP1 protein expression (FITC, green) and cell nuclei numbers (DAPI, blue). CD31- cells differentiated for 8 days in minimal differentiation medium (MDM) after exposure to rosiglitazone (1 µM) differentiate profoundly into brown adipocytes expressing high levels of UCP1 (FIG. 20A), whereas cells not exposed to rosiglitazone show a much lower level of differentiation and UCP1 expression (FIG. 20B). Cells maintained in proliferation medium (EGM-2) do not differentiate and express no UCP1 (FIG. 20C).
FIG. 21 shows BODIPY 500/510 C1, C12 fluorescence signal after brown adipocyte progenitor cells were induced to differentiate in culture for 9 days in various conditions (rosi and BMP-7 were used from day -3 to d0).
FIG. 22 shows fluorescence microscopy illustrating the BODIPY assay results for intracellular lipid droplet formation (BODIPY 500/510 C1, C12, green). CD31- cells were differentiated for 8 days in minimal differentiation medium (MDM) after exposure to rosiglitazone (1 µM) for 3 days (day -3 to day 0).

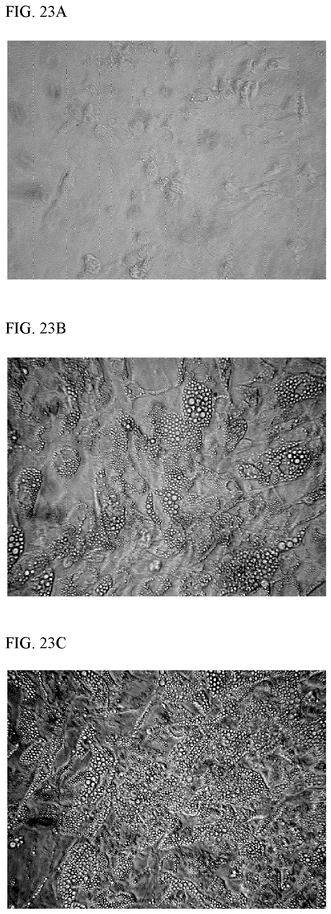

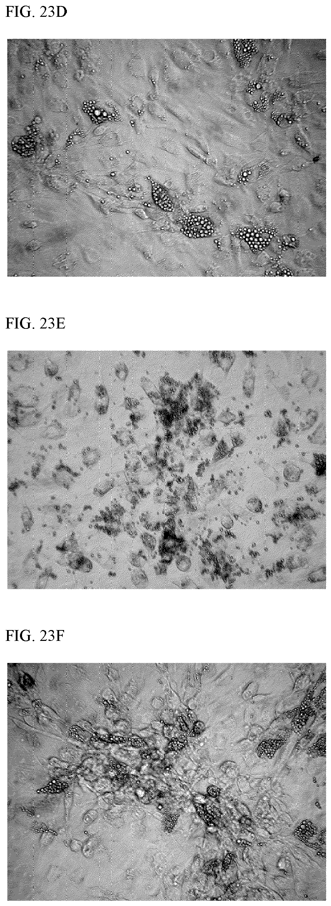

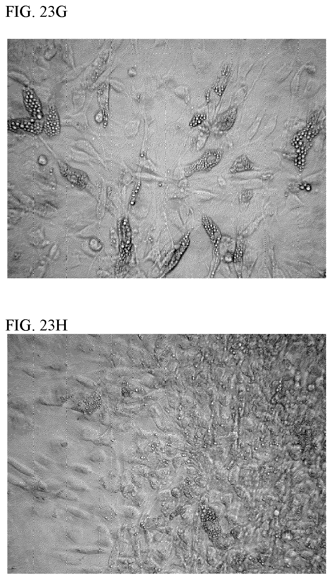

FIGS. 23A-23H show light microscopic photos of the CD31- cells and resulting brown adipocyte differentiation following treatment with several agents that promote brown adipocyte formation. FIG. 23A: Vehicle (DMSO). FIG. 23B: Rosiglitazone. FIG. 23C: BMP7. FIG. 23D: Diflunisal. FIG. 23E: Syrosingopine. FIG. 23F: Kaempferol. FIG. 23G: Probenecid. FIG. 23H: Tiapride.

### DETAILED DESCRIPTION

The present disclosure provides compositions for recruiting or producing brown adipocytes in vitro and in vivo from BAT progenitor cells found in human skeletal muscle. These agents, or combinations thereof, can be used to promote the differentiation of BAT progenitor cells into brown adipocytes and/or induce the expression of UCP1, FABP4 (aP2), PPARγ2, mtTFA, PGC-lα, and/or COX IV in BAT progenitor cells in vitro, in vivo, or both. Furthermore, these agents can be used to treat metabolic disease, including without limitation obesity, diabetes, insulin resistance, hyperlipidemia, and others conditions in a patient. The invention relates to an ex vivo method of promoting brown adipogenesis using an agent being Pituitary adenylate cyclase-activating polypeptide (PACAP) propeptide of 55 aa (aa 25-79).

The present disclosure is based, in part, on the discovery that various proteins, peptides, and small molecules (collectively, agents) play an important role in the differentiation of BAT progenitor cells. In particular, it has been found that the various agents disclosed herein markedly induce differentiation of BAT progenitor cells isolated from human skeletal muscle into mature, functional brown adipocytes. Treatment of these BAT progenitor cells with one or more of these various agents triggers commitment of these cells to brown adipocyte differentiation. In some cases, treatment with an agent for a period of time (e.g., a few hours, 1 day, 2 days, 3 days, or shorter or longer) prior to the introduction of an adipogenic medium results in brown adipocyte differentiation. In other cases, treatment with an agent contemporaneously with, and/or after the introduction of adipogenic medium results in brown adipocyte differentiation.

Since brown adipose tissue (BAT) is specialized for energy expenditure, the agents described herein are useful for the treatment of obesity and related disorders, such as diabetes. The agents can also be used to decrease fat stores in subjects including food animals, e.g., to improve the quality of the meat derived therefrom. The aspects described in par. [0035]-[0048] below are not part of the claimed invention.

Accordingly, in one aspect, the disclosure features an effective amount of an agent or combination of agents disclosed herein for use in a method of treating a subject, e.g., decreasing fat stores or weight in a subject such as a human.

In a further aspect, the disclosure features a population of agent-activated BAT progenitor cells for use in a method of treating a subject in need of decreasing fat stores or weigh, wherein said population of agent-activated progenitor cells undergo brown adipogenesis. The method can optionally include identifying a subject in need of decreasing fat stores or weight.

In a further aspect, the disclosure includes an agent and/or a population of agent-activated BAT progenitor cells for use in a method of enhancing insulin sensitivity in a subject, e.g., a subject that is insulin-resistant, wherein said population of agent-activated BAT progenitor cells undergo brown adipogenesis. The method can optionally include identifying a subject in need of enhanced insulin sensitivity.

In another aspect, the disclosure features an agent and/or a population of agent-activated BAT progenitor cells for use in a method of modulating brown adipose tissue function or development, e.g., promoting BAT adipogenesis, in a subject, wherein said population of agent-activated progenitor cells undergo brown adipogenesis.

In some aspects, methods described herein can include implanting a population of agent-activated BAT progenitor cells into a subject. The agent-activated cells can be implanted directly or can be administered in a scaffold, matrix, or other implantable device to which the cells can attach (examples include carriers made of, e.g., collagen, fibronectin, elastin, cellulose acetate, cellulose nitrate, polysaccharide, fibrin, gelatin, self-assembling small peptides, and combinations thereof). In general, the methods include implanting a population of agent-activated BAT progenitor cells comprising a sufficient number of cells to promote increased brown adipocyte mass in the subject, e.g., to increase the amount of brown adipocytes in the subject by at least 1%, e.g., 2%, 5%, 7%, 10%, 15%, 20%, 25% or more.

In some aspects, the methods include evaluating the level of BAT adipogenesis in a subject, by contacting isolated BAT progenitor cells from a subject with one or more of the agents disclosed herein. BAT differentiation can be evaluated by measuring any of, e.g., a BAT marker, such as uncoupling protein (UCP), e.g., UCP1, expression; BAT morphology (e.g., using visual, e.g., microscopic, inspection of the cells); or BAT thermodynamics, e.g., cytochrome oxidase activity, Na+-K+-ATPase enzyme units, or other enzymes involved in BAT thermogenesis.

In general, the subject can be a mammal. In some aspects, the subject is a human subject, e.g., an obese human subject. In some embodiments, the subject is a non-human mammal, e.g., an experimental animal, a companion animal, or livestock, e.g., a cow, pig, or sheep that is raised for food. Generally, where a protein or peptide is used to recruit brown adipocytes from BAT progenitor cells, the protein or peptide will be from the same or related species as the subject, e.g., human, cat, dog, cow, pig, or sheep. The protein or peptide can also be heterologous to the subject.

In some aspects, the methods include evaluating the subject for one or more of: weight, white adipose tissue stores, brown adipose tissue stores, adipose tissue morphology, insulin levels, insulin metabolism, glucose levels, thermogenic capacity, and cold sensitivity. The evaluation can be performed before, during, and/or after the administration of the agent and/or agent-activated BAT progenitor cells. For example, the evaluation can be performed at least 1 day, 2 days, 4 days, 7 days, 14 days, 21 days, 30 days or more or less before and/or after the administration.

In some aspects, the methods include one or more additional rounds of treatment with an agent or implantation of agent-activated BAT progenitor cells, e.g., to increase brown adipocyte mass, e.g., to maintain or further reduce obesity in the subject.

In some aspects where a protein or peptide agent is used, BAT progenitor cells can be genetically engineered to express increased levels of such protein or peptide, either stably or transiently. The cells can be, e.g., cultured mammalian cells, e.g., human cells. The expressed recombinant protein or peptide used will generally be of the same or realted species as the BAT progenitor cells, e.g., a human protein or peptide expressed in human cells. The recombinant protein or peptide can also be heterologous to the BAT progenitor cells.

In a further aspect, the present disclosure provides use of one or more agents disclosed herein, for promoting differentiation of BAT progenitor cells into brown adipocytes and/or inducing expression of UCP1, FABP4 (aP2), PPARγ2, mtTFA, PGC-lα, and/or COX IV in BAT progenitor cells in vitro.

Also provided herein is one or more agents disclosed herein, for use for the treatment of one or more diseases or conditions selected from overweight, obesity, insulin resistance, diabetes, hyperinsulinemia, hypertension, hyperlipidemia, hepatosteatosis, fatty liver, non-alcoholic fatty liver disease, hyperuricemia, polycystic ovarian syndrome, acanthosis nigricans, hyperphagia, endocrine abnormalities, triglyceride storage disease, Bardet-Biedl syndrome, Laurence-Moon syndrome, Prader-Willi syndrome, neurodegenerative diseases, and Alzheimer's disease. Methods for treating the foregoing diseases are also provided, comprising administering one or more agents disclosed herein to a subject in need thereof.

A further aspect relates to a pharmaceutical composition, comprising one or more agents disclosed herein, and a pharmaceutically acceptable excipient, diluent or carrier.

One particular aspect relates to use of an agent for recruiting brown adipocytes from BAT progenitor cells isolated from human skeletal muscle, wherein the agent is selected from one or more of:
an antihistamine such as Famotidine;
an antidopaminergic such as Tiapride hydrochloride or Thiethylperazine or Spiperone;
a ligand of tubulin such as Colchicine;
a Rauwolfia alkaloid or derivative such as Reserpine or Syrosingopine;
a potassium channel ligand such as Minoxidil;
an antagonist of calcium channels such as Felodipine;
Probenecid;
a derivative of prostaglandin F2 (PGF2) such as 9β,11α-PGF2 or 9α,11β-PGF2;
a peptide derived from Pituitary adenylate cyclase-activating polypeptide (PACAP) gene such as the PACAP Propeptide of 55 aa (aa 25-79);
a flavonoid such as kaempferol;
a fibroblast growth factor (FGF) such as FGF7 or FGF10 or FGF13;
a transient receptor potential melastatin 8 (TRPM8) ligand such as menthol or icilin;
bombesin;
stromal cell-derived factor 1(SDF-1) such as isoform SDF-1γ;
a cyclooxygenase inhibitor such as Diflunisal;
a biguanide such as Metformin;
a phosphodiesterase inhibitor such as a PDE3 inhibitor such as siguazodan;
a stimulator of soluble guanylate cyclase (sGC) such as riociguat;
b-type natriuretic peptide (BNP);
ciliary neurotrophic factor (CNTF);
interleukin-6 (IL-6);
orexin B; and
an α2 adrenergic receptor agonist such as Guanfacine hydrochloride.

The invention relates to an ex vivo method of promoting brown adipogenesis using an agent being PACAP Propeptide of 55 aa (aa 25-79) which is a peptide derived from Pituitary adenylate cyclase-activating polypeptide (PACAP) gene. The aspects described in par. [0050]-[0054] below are not part of the claimed invention.

In certain aspects, the agent is capable of inducing expression of UCP1, FABP4 (aP2), PPARγ2, mtTFA, PGC-lα, and/or COX IV in the BAT progenitor cells in vitro, in vivo, or both.

In some aspects, the agent can have one or more biological activities selected from the group consisting of:
(a) causing an increase or decrease in one or more of the following: Beta-3 adrenergic receptor (β3-AR), Solute carrier family 2, facilitated glucose transporter member 4 (SLC2A4), Elongation of very long chain fatty acids protein 3 (ELOVL3), CD36 antigen, Type II iodothyronine deiodinase (DIO2), BMP5, BMP6, FGF7, FGF10, FGF13, FGF21, Fatty acid binding protein 7 (FABP7), CXCL12, Atypical chemokine receptor 3 (ACKR3), Insulin-like growth factor-binding protein 4 (IGFBP4), Pituitary adenylate cyclase-activating polypeptide (PACAP), Adenylate cyclase 4 (ADCY4), Cell death activator CIDE-A (CIDEA), secreted frizzled-related protein 1 (SRFP1), SRFP2, brain-derived neurotrophic factor (BDNF), Vascular endothelial growth factor D (VEGF-D), Transforming growth factor beta-2 (TGFB2), cAMP-specific 3',5'-cyclic phosphodiesterase 4B (PDE4B), cAMP-specific 3',5'-cyclic phosphodiesterase 4D (PDE4D), High affinity cAMP-specific 3',5'-cyclic phosphodiesterase 7A (PDE7A), and cAMP-specific 3',5'-cyclic phosphodiesterase 7B (PDE7B);
(b) causing an increase in thermogenesis in brown adipose tissue and/or skeletal muscle tissue;
(c) causing an increase in insulin sensitivity of skeletal muscle, white adipose tissue, or liver;
(d) causing an increase in glucose tolerance;
(e) causing an increase in basal respiration, maximal respiration rate, or uncoupled respiration;
(f) causing an increase in metabolic rate; and
(g) causing a decrease in hepatosteatosis.

In certain aspects, the agent can cause an increase or decrease in one or more of the following: Beta-3 adrenergic receptor (β3-AR), Solute carrier family 2, facilitated glucose transporter member 4 (SLC2A4), Elongation of very long chain fatty acids protein 3 (ELOVL3), CD36 antigen, and Type II iodothyronine deiodinase (DIO2).

In some aspects, the agent is capable of modulating a metabolic response in a subject or preventing or treating a metabolic disorder in a subject. The metabolic disorder can, in some embodiments, be one or more of obesity, type II diabetes, insulin resistance, hyperinsulinemia, hypertension, hyperlipidemia, hepatosteatosis, fatty liver, non-alcoholic fatty liver disease, hyperuricemia, polycystic ovarian syndrome, acanthosis nigricans, hyperphagia, endocrine abnormalities, triglyceride storage disease, Bardet-Biedl syndrome, Laurence-Moon syndrome, Prader-Willi syndrome, neurodegenerative diseases, and Alzheimer's disease.

Another aspect relates to an agent selected from one or more of agents disclosed herein for use in a method of promoting brown adipogenesis in a subject in need thereof. The method can further include modulating a metabolic response in the subject and/or preventing or treating a metabolic disorder in the subject. The metabolic disorder may be one or more of obesity, type II diabetes, insulin resistance, hyperinsulinemia, hypertension, hyperlipidemia, hepatosteatosis, fatty liver, non-alcoholic fatty liver disease, hyperuricemia, polycystic ovarian syndrome, acanthosis nigricans, hyperphagia, endocrine abnormalities, triglyceride storage disease, Bardet-Biedl syndrome, Laurence-Moon syndrome, Prader-Willi syndrome, neurodegenerative diseases, and Alzheimer's disease. In certain aspects, the method further includes contacting a cell of the subject with the agent, and optionally transplantation of said cell into the subject after said contacting step. The cell in some aspects can be a BAT progenitor cell isolated from human skeletal muscle. The cell can be positive for CD34 and/or negative for CD31.

As used herein, "agent-activated" means that the BAT progenitor cell or cells have been treated with one or more agents as described herein and are at least partially committed to differentiate into brown adipocytes. The cells can be autologous, allogeneic, or xenogeneic. "Brown adipogenesis" means generation of brown adipocytes from BAT progenitor cells in vivo, in vitro, or partially in vivo and partially in vitro. It should be noted that brown adipogenesis may be induced, i.e., the so-called "BAT progenitor cells" before induction by, e.g., one or more agents disclosed herein, was not necessarily committed to differentiate into brown adipocytes and may be reprogrammed or transdifferentiate into brown adipocytes from a stem cell or a somatic cell. "Recruiting brown adipocytes" means promoting or enhancing differentiation of BAT progenitor cells into brown adipocytes, and/or increasing the amount or concentration of brown adipocytes, in vivo and/or in vitro.

Provided (but not claimed) herein are agents (e.g., compounds, proteins, biologicals, and the like) that can promote the differentiation of BAT progenitor cells into brown adipocytes and/or induce the expression of the UCP1 gene in vitro, in vivo, or both. Such agents can be identified by screening compounds, proteins, biologicals, and the like. For example, BAT progenitor cells (e.g., those isolated from human skeletal muscle) can be used to screen agents for the ability to induce expression of the UCP1 gene and/ or differentiation of the BAT progenitor cells into brown adipocytes. Agents identified in this manner can be used for a variety of research, diagnostic and therapeutic purposes, including, for example, treatment of metabolic diseases such as obesity, type 2 diabetes, insulin-resistance, dyslipidemia, and the like. In some aspects, an agent identified by an assay according to the present disclosure is optimized for improvement of its physico-chemical and/ or pharmacokinetic properties.

Expression of UCP1, FABP4 (aP2), PPARγ2, mtTFA, PGC-lα, and/or COX IV in BAT progenitor cells in vitro and in vivo can be enhanced according to methods provided in the present disclosure. In some aspects, exposure to adipogenic media can be used to stimulate increased expression of UCP1, FABP4 (aP2), PPARγ2, mtTFA, PGC-lα, and/or COX IV in BAT progenitor cells. The aspects described in par. [0058]-[0065] below are not part of the claimed invention.

Accordingly, in some aspects, the following agents, or combinations thereof, can be used to promote the differentiation of BAT progenitor cells into brown adipocytes and/or induce the expression of UCP1, FABP4 (aP2), PPARγ2, mtTFA, PGC-lα, and/or COX IV in BAT progenitor cells in vitro, in vivo, or both: a PDE3 inhibitor (e.g., siguazodan), a PDE4 inhibitor (e.g., rolipram), a derivative of prostaglandin F2 (PGF2) such as 9β,11α-prostaglandin F2 or 9α,11β-prostaglandin F2, apepdide derived (e.g., a portion) from the Pituitary adenylate cyclase-activating polypeptide (PACAP, ADCYAP1, UniProt P18509) gene such as the PACAP Propeptide of 55 aa (aa 25-79), BDNF (brain-derived neurotrophic factor), a TGR5 agonist such as oleanolic acid, BMP-7, a flavonoid such as kaempferol (KMP, CAS number 520-18-3), a stimulator of soluble guanylate cyclase (sGC) such as riociguat (BAY 63-2521, CAS 625115-55-1), fibroblast growth factors (such as FGF7 (fibroblast growth factor-7, KGF, keratinocyte growth factor), FGF10 (fibroblast growth factor-10, KGF-2, keratinocyte growth factor-2), or FGF13 (fibroblast growth factor-13)), BNP (b-type natriuretic peptide), a TRPM8 (CMRI) ligand such as menthol or icilin, a bombesin peptide such as from the toad (UniProt P84214), CNTF (ciliary neurotrophic factor, UniProt P05231), interleukin-6 (IL-6), orexin B, SDF-1γ (CXCL12), or Guanfacine hydrochloride.

Further, in other aspects, additional agents or combinations thereof that can be used to promote the differentiation of BAT progenitor cells into brown adipocytes and/or induce the expression of UCP1 include prostaglandin J2 (PGJ2), 24(S)-Hydroxycholesterol, forms of vitamin D such as 1,25-Dihydroxyvitamin D3 or 24,25-Dihydroxyvitamin D3, and a cyclooxygenase inhibitor such as Diflunisal.

In still other aspects, additional agents or combinations thereof that can be used to promote the differentiation of BAT progenitor cells into brown adipocytes and/or induce the expression of UCP1 include bone morphogenetic proteins such as BMP5 (bone morphogenetic protein 5, UniProt P22003) and BMP6 (bone morphogenetic protein 6, UniProt P22004), Platelet-derived growth factor receptor-like protein (PDGFRL, UniProt Q15198), Vascular endothelial growth factor D (VEGF-D, FIGF, UniProt 043915), CYTL1 (cytokine-like protein 1, UniProt Q9NRR1), SCG2 (secretogranin-2, UniProt P13521), NPTX2 (neuronal pentraxin-2, UniProt P47972), OLFML2B (olfactomedin-like protein 2B, UniProt Q68BL8), TFPI2 (tissue factor pathway inhibitor 2, UniProt P48307), IFNE (interferon epsilon, UniProt Q86WN2), a Prostaglandin F2-a receptor (PTGFR, prostanoid FP receptor, UniProt P43088) ligand such as prostaglandin F2, CNTF (ciliary neurotrophic factor), Interleukin-6 (IL-6, UniProt P05231), Interleukin-15 (IL-15, UniProt P40933), CXCL12 ((chemokine (C-X-C motif) ligand 12), stromal cell-derived factor 1, SDF1, UniProt P48061 isoform SDF-1g/UniProt P48061-3), and/or a ligand of Atypical chemokine receptor 3 (ACKR3, CMKOR1, CXCR7, GPR159, RDC1, UniProt P25106) such as SDF1 (CXCL12).

In still further aspects, other agents or combinations thereof that can be used to promote the differentiation of BAT progenitor cells into brown adipocytes and/or induce the expression of UCP1 include a biguanide such as Metformin, an antihistamine such as Famotidine, an antidopaminergic such as Tiapride hydrochloride, Spiperone, Thiethylperazine, a microtubule modulator such as Colchicine, a Rauwolfia alkaloid or derivative of such as Reserpine or Syrosingopine, a potassium channel ligand such as Minoxidil, Probenecid, or a calcium channel antagonist such as Felodipine.

In some aspects, treatment of a subject, including a human subject, with one or a combination of agents shown here, results in an increase in the production of UCP1 mRNA or protein in the subject's skeletal muscle. For example, treatment of subjects with rosiglitazone can induce the appearance or differentiation of brown adipocytes in skeletal muscle, enhance expression of the UCP1 gene in existing brown adipocytes in or near skeletal muscle (between myofibers, at the surface of and/or adjacent to skeletal muscle tissue), or both. In some aspects, the appearance or differentiation of brown adipocytes in skeletal muscle can be induced in a subject suffering from a metabolic disease. The brown adipocytes can provide a glucose sink with high mitochondrial and cellular respiration and fatty acid oxidation rates, dissipating energy as heat (uncoupled oxidative phosphorylation). The subject metabolic rate can be enhanced, and a decrease in body weight can be induced. Induction of the appearance or differentiation of brown adipocytes can also yield improvements in insulin sensitivity, blood glucose homeostasis and cardiovascular disease risk factors. Brown adipocytes may further secrete factors that contribute to reaching a healthy energy balance and low body fat levels, increased insulin sensitivity and improved blood glucose homeostasis or cardiovascular health.

Accordingly, in some aspects, the agents disclosed herein, or combinations thereof, can be used for treatment of a subject, including a human subject. In some aspects, these agents may promote the differentiation of BAT progenitor cells into brown adipocytes. In other aspects these agents may induce the expression of UCP1, FABP4 (aP2), PPARγ2, mtTFA, PGC-lα, and/or COX IV in BAT progenitor cells in vitro, in vivo, or both.

In some aspects, the treated metabolic disease may be obesity, type II diabetes, insulin resistance, hyperinsulinemia, hypertension, hyperlipidemia, hepatosteatosis, fatty liver, non-alcoholic fatty liver disease, hyperuricemia, polycystic ovarian syndrome, acanthosis nigricans, hyperphagia, endocrine abnormalities, triglyceride storage disease, Bardet-Biedl syndrome, Laurence-Moon syndrome, Prader-Willi syndrome, neurodegenerative diseases, and Alzheimer's disease.

In other aspects, agents may be used to activate isolated, autologous BAT progenitor cells that are then used for treatment of a subject, including a human subject.

### Identification of Molecular Pathways

Gene chip studies were performed to identify molecular pathways that play a role in the differentiation of CD31- progenitor cells into brown adipocytes and/or the induction of the expression of UCP1. CD31- cells were isolated from human skeletal muscle biopsies as described previously in WO2013071063, and were used in two studies: (1) cAMP study: CD31- cells were differentiated as described in WO2013071063 (Control) plus addition of vehicle (Control 1 sample) or cAMP (cAMP sample); and (2) Rosiglitazone study: CD31- cells were differentiated as described previously in WO2013071063 except that rosiglitazone was omitted from the adipogenic medium (Control 2 sample). Rosiglitazone was added only to the second sample (Rosiglitazone sample) in this study. As discussed above, these agents have been shown to promote the differentiation of CD31- cells into brown adipocytes and the expression of UCP1.

Total RNA was purified from these samples, and transcriptional profiles were assessed with Illumina Human WG-6 BeadChip (Expression Analysis, Inc., Durham, NC). Results were analyzed with Ingenuity Pathway Analysis 7.0 (trial version). These results were used to determine what molecular pathways are involved in the differentiation of CD31- cells into brown adipocytes, and, more importantly, what molecular targets can be used for the development of agents that promote the appearance of brown adipocytes and the expression of UCP1.

Based on this work, the following mechanisms and agents were found to promote brown adipocyte development from BAT progenitor cells: a PPARγ ligand (e.g., rosiglitazone), a PDE3 inhibitor (e.g., siguazodan), a PDE4 inhibitor (e.g., rolipram), BMP7 (bone morphogenetic protein 7, UniProt P18075), BMP5 (bone morphogenetic protein 5, UniProt P22003), BMP6 (bone morphogenetic protein 6, UniProt P22004), FGF7 (fibroblast growth factor-7, KGF, keratinocyte growth factor), FGF10 (fibroblast growth factor-10, KGF-2, keratinocyte growth factor-2), BNP (b-type natriuretic peptide), FGF13 (fibroblast growth factor-13), BDNF (brain-derived neurotrophic factor), a stimulator of soluble guanylate cyclase (sGC) (e.g., riociguat (BAY 63-2521, CAS 625115-55-1), a TRPM8 (CMRI) ligand (e.g., menthol, icilin), Platelet-derived growth factor receptor-like protein (PDGFRL, UniProt Q15198), Vascular endothelial growth factor D (VEGF-D, FIGF, UniProt 043915), CYTL1 (cytokine-like protein 1, UniProt Q9NRR1), SCG2 (secretogranin-2, UniProt P13521), NPTX2 (neuronal pentraxin-2, UniProt P47972), OLFML2B (olfactomedin-like protein 2B, UniProt Q68BL8), TFPI2 (tissue factor pathway inhibitor 2, UniProt P48307), IFNE (interferon epsilon, UniProt Q86WN2), and a Prostaglandin F2-a receptor (PTGFR, prostanoid FP receptor, UniProt P43088) ligand such as prostaglandin F2. Still other mechanisms/agents that were found to promote brown adipocyte development from BAT progenitor cells based on gene chip data include: a peptide derived from the Pituitary adenylate cyclase-activating polypeptide (PACAP, ADCYAP1, UniProt P18509) gene such as PACAP Propeptide of 55 aa (aa 25-79) (200 nM-2 µM), CNTF (ciliary neurotrophic factor), Interleukin-6 (IL-6, UniProt P05231), Interleukin-15 (IL-15, UniProt P40933), CXCL12 (chemokine (C-X-C motif) ligand 12), stromal cell-derived factor 1 (SDF1, UniProt P48061) isoform SDF-1g/UniProt P48061-3), and/or a ligand of Atypical chemokine receptor 3 (ACKR3, CMKOR1, CXCR7, GPR159, RDC1, UniProt P25106) such as SDF1 (CXCL12). Only Pituitary adenylate cyclase-activating polypeptide (PACAP) propeptide of 55 aa (aa 25-79) is used according to the invention.

### Screening of potential modulators of human UCP1 mRNA

CD31- cells can be used as a tool to identify agents (e.g., compounds, proteins, biologicals, and the like) that induce the differentiation of these cells into brown adipocytes or modulate the expression of UCP1. For example, an RT-PCR based approach can be used to measure UCP1 mRNA levels which may be affected by certain agents.

This allows the identification of agents that can enhance the differentiation of CD31- cells into brown adipocytes and/or the expression of UCP1 by enhancing the transcription of the UCP1 gene and/or by stabilizing the UCP1 transcript.

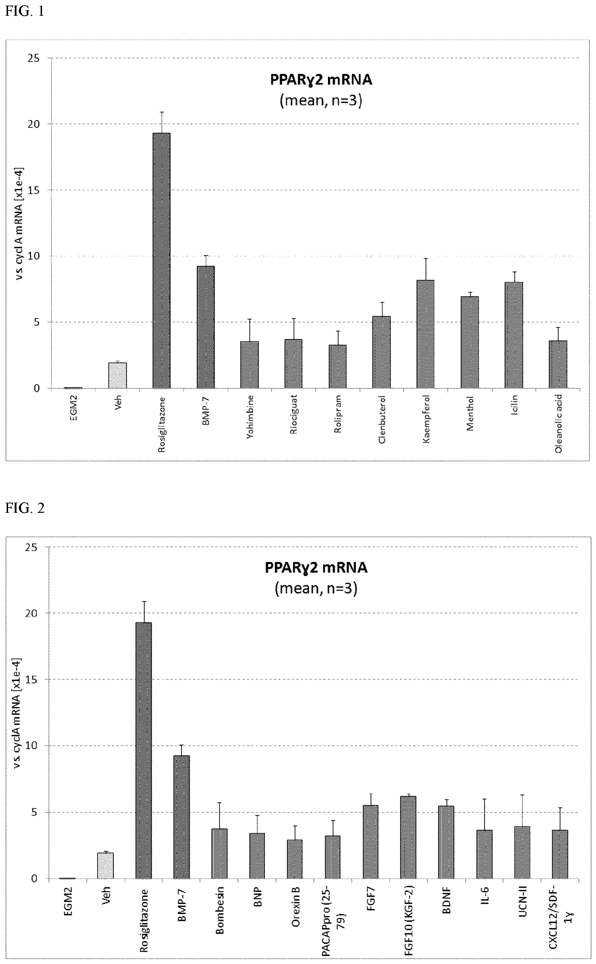

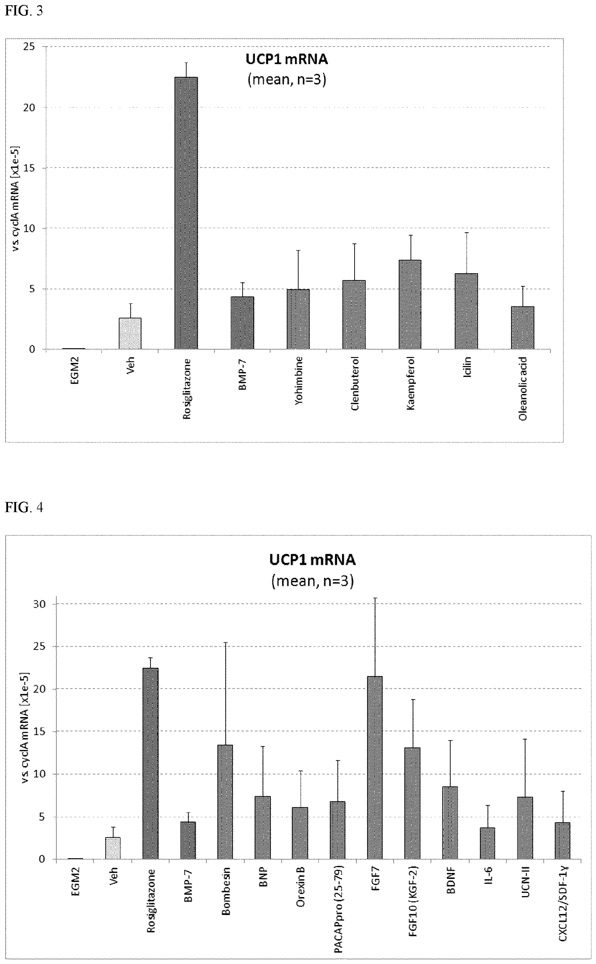

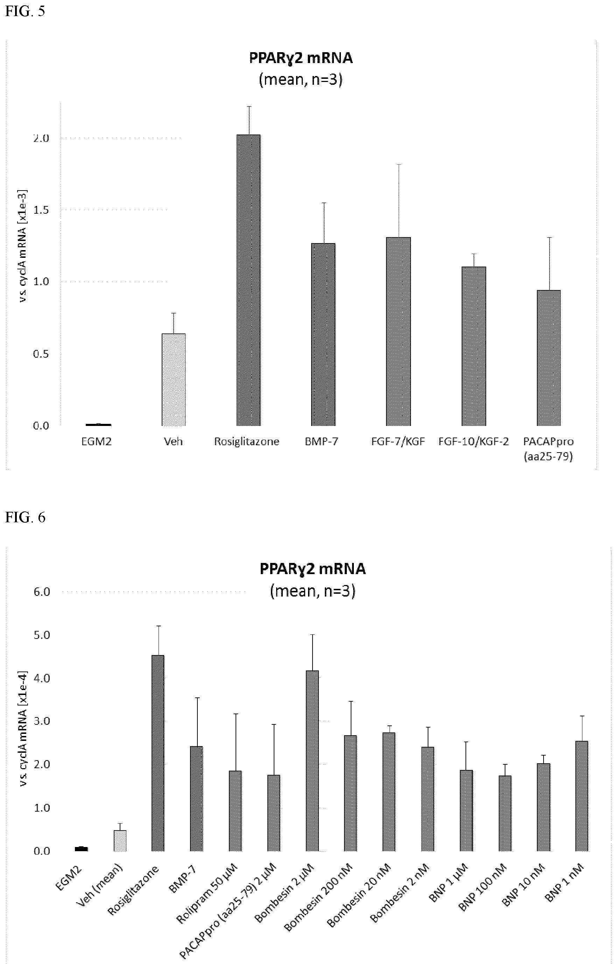

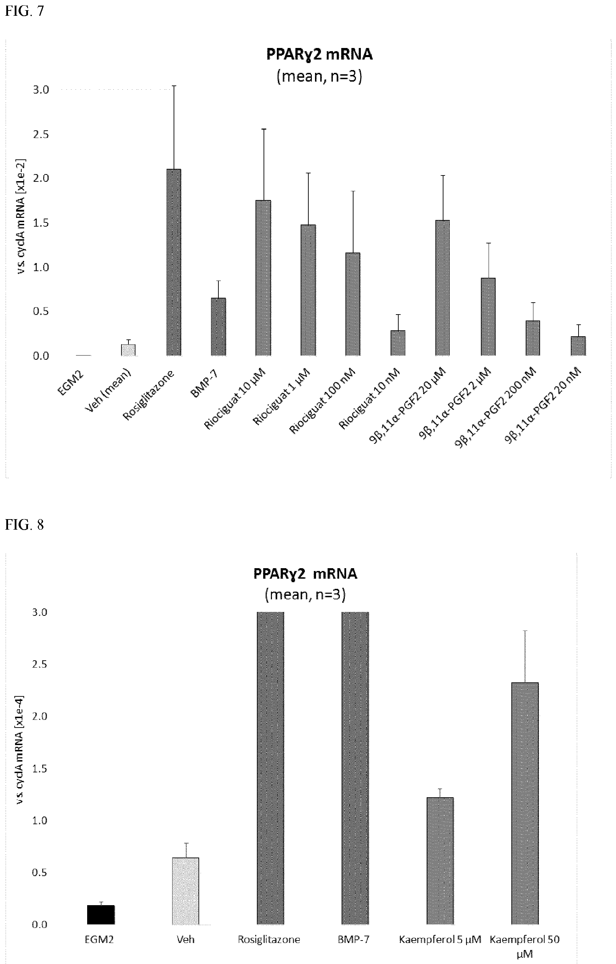

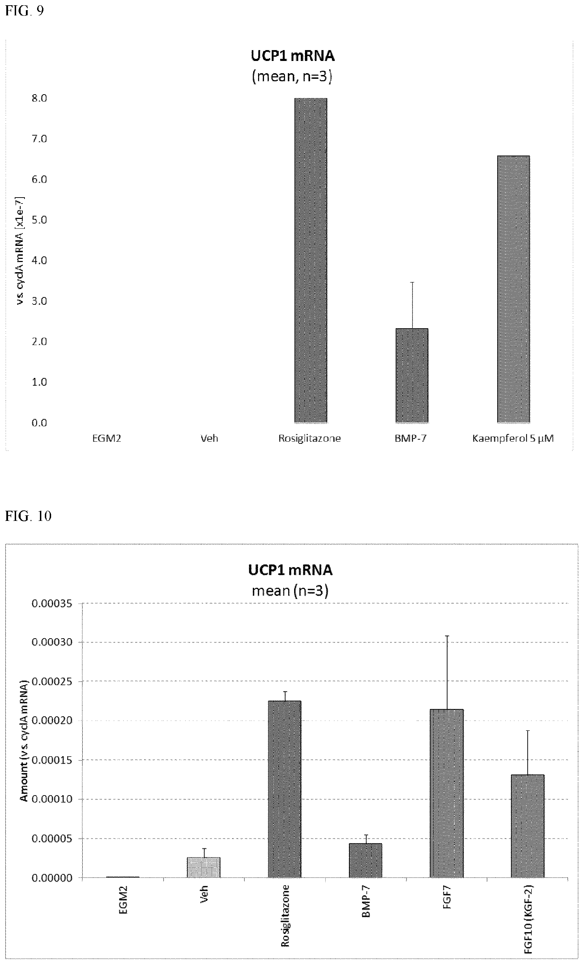

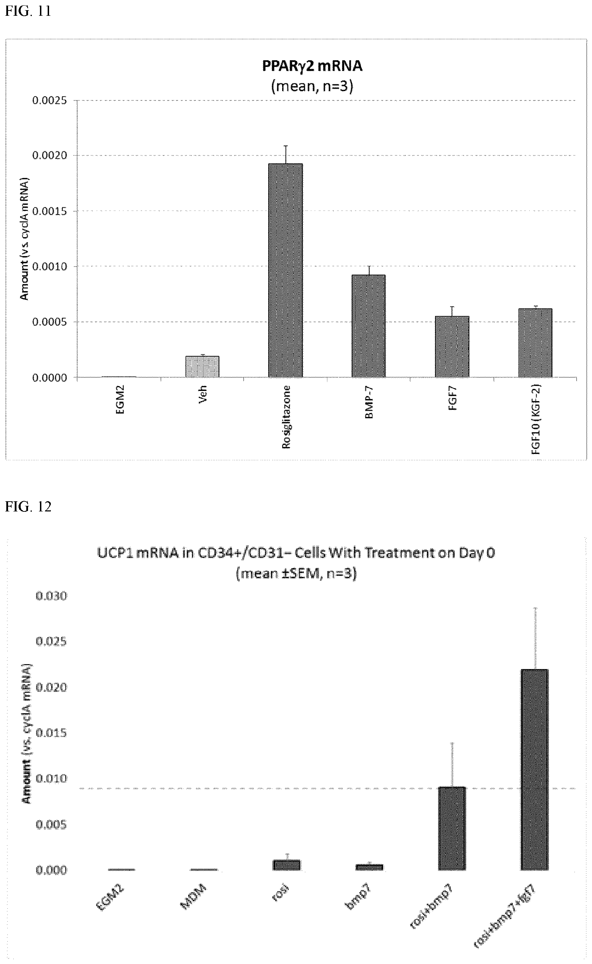

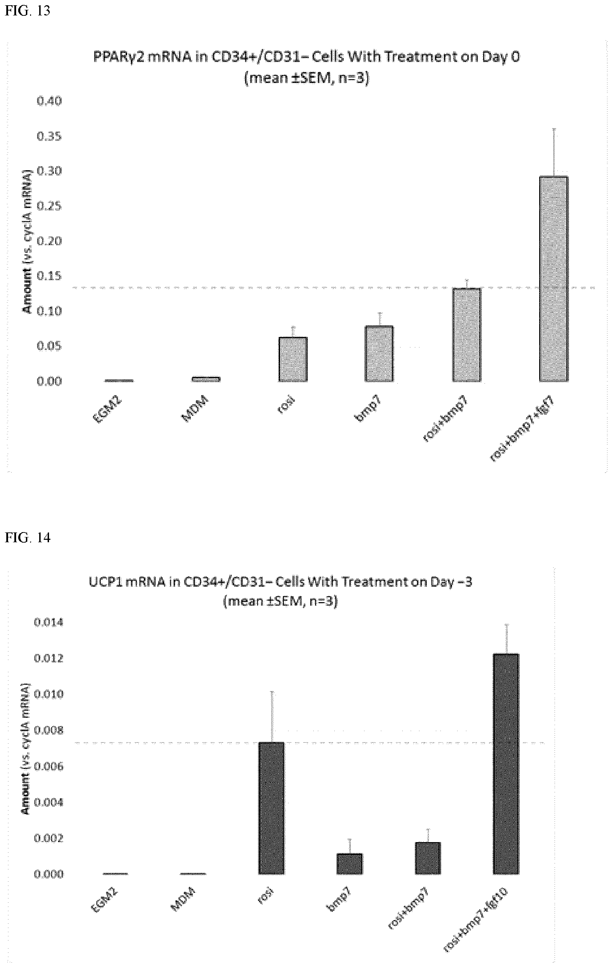

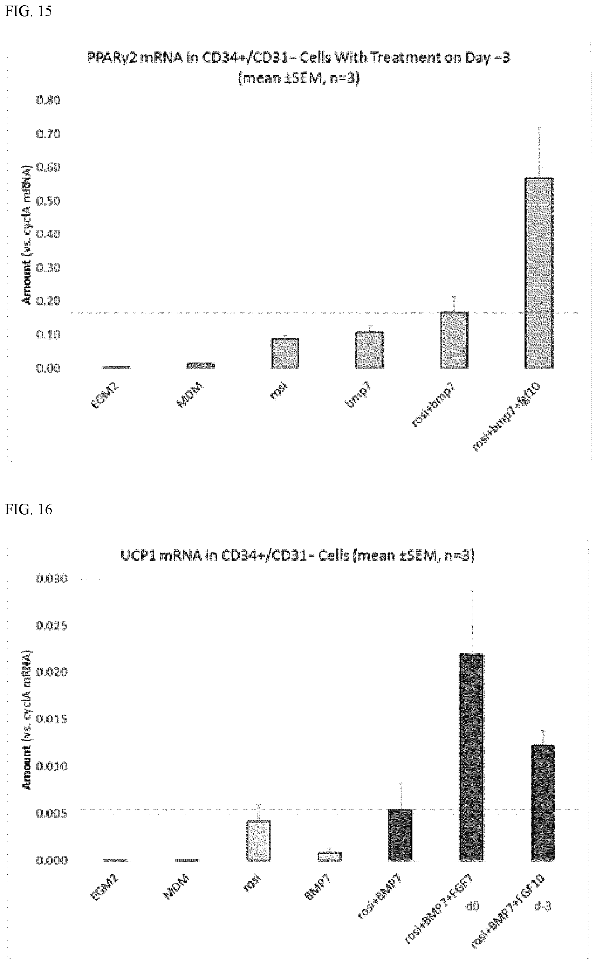

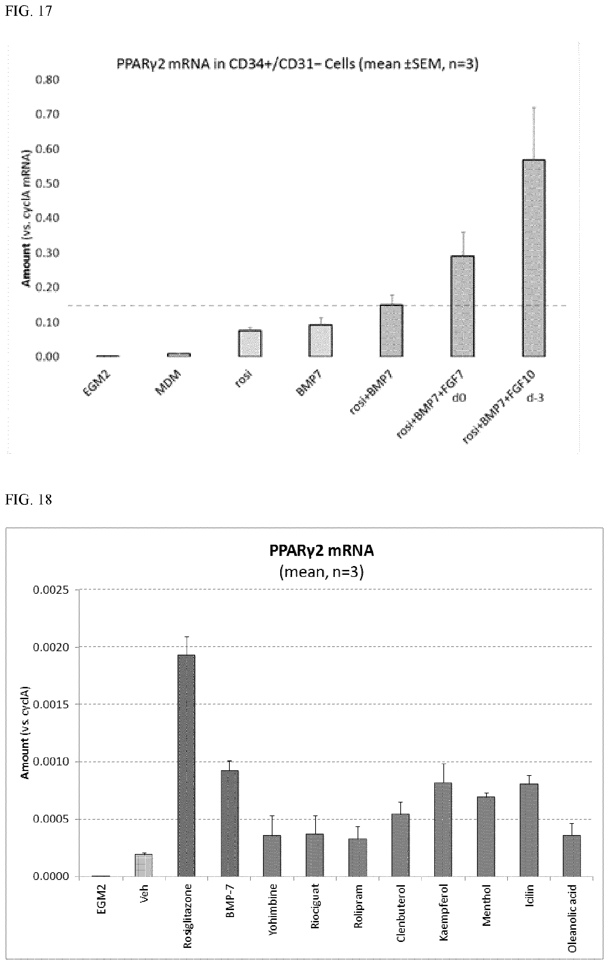

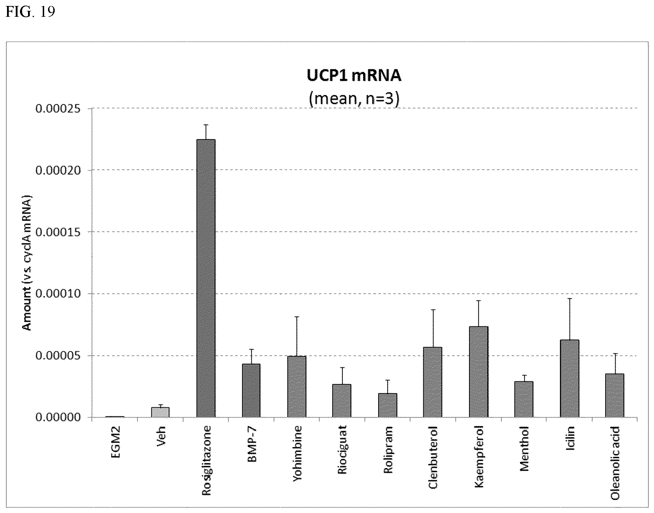

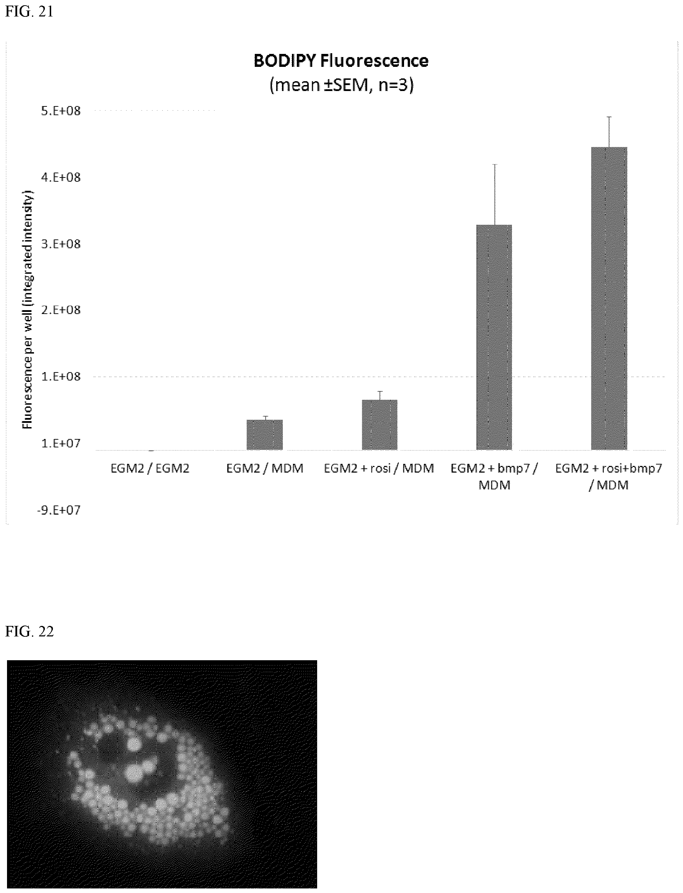

For example, a PPARγ ligand like rosiglitazone can be used to promote the differentiation of CD31-progenitor cells into brown adipocytes (FIGS. 1-19, 20A, 21, 22). Another example is the use of the recombinant protein, human BMP-7 (FIGS. 1-19, 21).

A robust method, previously disclosed in WO2013071063 and incorporated herein by reference, was used for detection of CD31- cell differentiation into brown adipocytes by simultaneously quantifying mRNA species corresponding to the brown adipocyte marker UCP1, the adipocyte marker PPARγ2, and the "housekeeping" gene cyclophilin A which was used as the internal control.

This method permits analysis of a large number of samples to identify agents that enhance the differentiation of CD31- cells into brown adipocytes. When differentiated into brown adipocytes CD31-cells express much higher levels of UCP1 and PPARy2 mRNA for a given level of cyclophilin A. UCP1 and PPARy2 mRNA levels normalized to cyclophilin A mRNA levels give an indication of the level of differentiation of the CD31- cells into brown adipocytes, independent of the total number of cells in the sample.

Quantification of UCP1, PPARy2 and cyclophilin A mRNA by multiplexed TaqMan real-time PCR was thus used to quantify differentiation of the CD31- cells into brown adipocytes.

Using this method the following agents were identified or confirmed to promote the differentiation of BAT progenitor cells into brown adipocytes and/or induce the expression of UCP1, FABP4 (aP2), PPAPγ2, mtTFA, PGC-lα, and/or COX IV in BAT progenitor cells in vitro, in vivo, or both: a PPARγ ligand like rosiglitazone, a PDE3 inhibitor like siguazodan, a PDE4 inhibitor like rolipram, a derivative of prostaglandin F2 (PGF2) like 9β,11α-prostaglandin F2, a pepdide derived from the Pituitary adenylate cyclase-activating polypeptide (PACAP, ADCYAP1, UniProt P18509) gene like the PACAP Propeptide of 55 aa (aa 25-79), BDNF (brain-derived neurotrophic factor), a TGR5 agonist like oleanolic acid, BMP-7, kaempferol (KMP, CAS number 520-18-3), a stimulator of soluble guanylate cyclase (sGC) like riociguat (BAY 63-2521, CAS 625115-55-1), FGF7 (fibroblast growth factor-7, KGF, keratinocyte growth factor), FGF10 (fibroblast growth factor-10, KGF-2, keratinocyte growth factor-2), BNP (b-type natriuretic peptide), a TRPM8 (CMRI) ligand like menthol or icilin, bombesin, CNTF (ciliary neurotrophic factor), interleukin-6 (IL-6), orexin B, SDF-1γ (CXCL12), and FGF13 (fibroblast growth factor-13). Only Pituitary adenylate cyclase-activating polypeptide (PCAP propeptide of 55 aa (aa 25-79) is used according to the invention.

Except otherwise indicated, all organic and inorganic chemicals of analytical or molecular biology grade were purchased from Sigma Chemical Co. (St Louis, MI), Life Technologies (Grand Island, NY), GenScript, Prospec, LifeTein, AnaSpec. Rosiglitazone was purchased from Cayman Chemical (# 71742) and recombinant human BMP7 (rhBMP7) was from R&D Systems (100 µg/ml, 6.3 µM, # 354-BP-010).

### Cell culture

Cells were seeded at 10,000 per cm² in 0.2% gelatin coated plates (48-well tissue culture, Chemglass # CLS-3500-048), cultured until confluency (2-4 days) at 37°C in Endothelial cell growth medium-2 (EGM2) (BulletKit growth medium, Lonza # CC-3162) and until differentiation (10-16 more days). After 2 or 3 days in EGM2 medium the cells were induced to differentiate by replacing the medium with an adipogenic medium, which is a modification of the adipogenic medium described by Rodriguez et al. [21] and may or may not contain a differentiation inducing agent (e.g., PPARγ agonist). The MDM described above contains: DMEM/Ham's F-12 50/50 Mix (3.151 g/l, 17.5 mM D-glucose, 3.651g/l L-glutamine) (Cellgro # 10-090-CV), 5 µg/ml (0.86 µM) insulin, 1 µM dexamethasone, 100 µM 3-isobutyl-1-methylxanthine, 0.2 nM 3,3',5-triiodo-L-thyronine, 10 µg/ml (127 nM) transferrin, and 1% penicillin-streptomycin. If rosiglitazone is used as a differentiation-inducing agent, it can be supplied at 1µM or any other concentration sufficient to induce differentiation of BAT progenitor cells into adipocytes.

For cell expansion studies, confluent cells grown in EGM2 medium only were detached by treatment with trypsin-EDTA for 3-5 min at 37°C, and then split 1:3 or 1:4 and cultured as described above.

### Quantification of UCP1 and PPARy2 mRNA by quantitative reverse transcription, real-time PCR

Total RNA was prepared from cells using PureLink RNA Isolation Kit (Invitrogen # 12183-016) First strand cDNA were synthesized using the High Capacity cDNA Reverse Transcription kit (Applied Biosystems, Foster City, CA) and random primers.

Quantitative real-time PCR was performed using an Applied Biosystems StepOnePlus^{™} instrument, TaqMan Gene Expression Master Mix (Applied Biosystems # 4369016), and custom TaqMan gene expression probes and primers for human uncoupling protein-1 "UCP1" (GenBank NM_021833) and for human peptidylprolyl isomerase A "cyclophilin A" (GenBank NM_021130) . Custom TaqMan Gene Expression reagents were also developed for simultaneous measurement of peroxisome proliferator-activated receptor gamma, transcript variant 2 (PPARγ2) (GenBank NM_015869) in a multiplexed fashion (with UCP1 and cyclophilin A): UCP1 FAM-MGB probe: TCA AGG GGT TGG TAC CTT CC (SEQ ID NO.: 1), sense primer: CAC TAA CGA AGG ACC AAC GG (SEQ ID NO.: 2), and antisense primer: TTC CAG GAT CCA AGT CGC AA (SEQ ID NO.: 3). Cyclophilin A NED-MGB probe: ACT GCC AAG ACT GAG TGG TT (SEQ ID NO.: 4), sense primer: CAA ATG CTG GAC CCA ACA CA (SEQ ID NO.: 5), and antisense primer: TCA CTT TGC CAA ACA CCA CA (SEQ ID NO.: 6). PPARg2 VIC-MGB probe: TCA CAA GAA ATG ACC ATG GTT G (SEQ ID NO.: 7), sense primer: AGC GAT TCC TTC ACT GAT ACA C (SEQ ID NO.: 8), and antisense primer: CCA GAA TGG CAT CTC TGT GT (SEQ ID NO.: 9).

Cyclophilin A was used as a control to account for any variations due to the efficiency of reverse transcription. Arbitrary units were determined by normalizing target mRNA levels to cyclophilin A mRNA levels (based on Cts).

### Statistical analysis

Data are expressed as means ± SEM. Significances were evaluated using the unpaired Student's t-test. Significances were set at p<0.05.

### Pictures for cell morphology

Pictures of cells were taken using a hand-held digital camera (Nikon Coolpix 950) and inverted microscope (Nikon TMS) used for cell culture observations; images were optimized using Adobe Photoshop Elements 8 functions for Auto Contrast and Auto Levels.

### Quantification of UCP1 protein

Differentiation of brown adipocyte progenitors into brown adipocytes can be detected through quantification of UCP1 protein by immunohistochemistry (IHC).

Culturing and differentiation of CD31- cells into brown adipocytes were performed using adipogenic differentiation medium lacking (Minimal Differentiation Medium, MDM) or containing 1 µM rosiglitazone (Reference Differentiation Medium, RDM). After 15 days of differentiation cells were fixed with 4% Paraformaldehyde PBS pH 7.4, and incubated with a UCP1 antibody (Abcam ab23841) and Alexafluor 488 goat anti-rabbit antibody to quantify relative UCP1 levels (green) according to standard protocols. Prior to fixation of cells, nuclei were labeled with 5 µM DAPI (blue) for 10 minutes. Each treatment condition was evaluated in triplicate in a 96-well plate corresponding to approximately 360-480 cells for each data point in total. The InCell 1000 Developer Toolbox software was used to develop an automated cell detection script to measure UCP1 signal intensity, using the nuclei and cytoplasm detection algorithms. As a readout, total intensity of UCP1 signal within the cell was used, normalized to cell number.

In some aspects, agents or combinations thereof that were identified using this technique include Famotidine, Tiapride hydrochloride, Guanfacine hydrochloride, Reserpine, Minoxidil, Spiperone, Diflunisal, Syrosingopine, Probenecid, Metformin, Thiethylperazine, Colchicine, and Felodipine.

### Detection of brown adipocyte differentiation

BODIPY fluorescent dye-labeled neutral lipids become incorporated in cytoplasmic lipid droplets allowing analysis of cellular fatty acid uptake and adipocyte differentiation by fluorescent cellular imaging. Cells are incubated with C1-BODIPY^{®} 500/510 C12 (Molecular Probes # D-3823) for 3 to 6 hours before imaging on a microplate-based high-throughput, high-content, brightfield and fluorescence cellular imager and analyzer (Cyntellect Celigo^{®} or GE Healthcare IN Cell Analyzer).

### References

[1] M. Klingenspor, Cold-induced recruitment of brown adipose tissue thermogenesis., Exp Physiol. 88 (2003) 141-148.
[2] B. Cannon, J. Nedergaard, The biochemistry of an inefficient tissue: brown adipose tissue, Essays Biochem. 20 (1985) 110-164.
[3] N.J. Rothwell, M.J. Stock, A role for brown adipose tissue in diet-induced thermogenesis, Nature. 281 (1979) 31-35.
[4] B. B. Lowell, V. S-Susulic, A. Hamann, J.A. Lawitts, J. Himms-Hagen, B. B. Boyer, L.P. Kozak, J. S. Flier, Development of obesity in transgenic mice after genetic ablation of brown adipose tissue, Nature 366 (1993) 740-742.
[5] H. M. Feldmann, V. Golozoubova, B. C. M. Cannon, J. Nedergaard, UCP1 ablation induces obesity and abolishes diet-induced thermogenesis in mice exempt from thermal stress by living at thermoneutrality, Cell Metab. 9 (2009) 203-209.
[6] J. Kopecky, G. Clarke, S. Enerback, B. Spiegelman, L.P. Kozak, Expression of the mitochondrial uncoupling protein gene from the aP2 gene promoter prevents genetic obesity, J Clin Invest. 96 (1995).
[7] K. Tsukiyama-Kohara, F. Poulin, M. Kohara, CT. DeMaria, A. Cheng, Z. Wu, A. C. Gingras, A. Katsume, M. Elchebly, B. M. Spiegelman, M. E. Harper, M. L. Tremblay, N. Sonenberg, Adipose tissue reduction in mice lacking the translational inhibitor 4E-BP1, Nature Medicine 7 (2001) 1128-1132.
[8] K. Almind, M. Manieri, W.I. Sivitz, S. Cinti, CR. Kahn, Ectopic brown adipose tissue in muscle provides a mechanism for differences in risk of metabolic syndrome in mice, Proc Natl Acad Sci USA. (2007).
[9] M. Del Mar Gonzalez-Barroso, D. Ricquier, A.M. Cassard-Doulcier, The human uncoupling protein- 1 gene (UCP1): present status and perspectives in obesity research, Obes Rev. 1 (2000) 61-72.
[10] B. Cannon, J. Nedergaard, Brown adipose tissue: function and physiological significance, Physiol Rev. 84 (2004) 277-359.
[11] W.D. van Marken Lichtenbelt, J.W. Vanhommerig, N. M. Smulders, J. M. Drossaerts, G.J. Kemerink, N. D. Bouvy, P. Schrauwen, G.J. Teule, Cold-activated brown adipose tissue in healthy men, N Engl J Med. 360 (2009) 1500-1508.
[12] A.M. Cypess, S. Lehman, G. Williams, I. TaI, D. Rodman, A.B. Goldfine, F.C Kuo, E.L. Palmer, Y.H. Tseng, A. Doria, G.M. Kolodny, CR. Kahn, Identification and importance of brown adipose tissue in adult humans, N Engl J Med. 360 (2009) 1509-1517.
[13] K.A. Virtanen, M.E. Lidell, J. Orava, M. Heglind, R. Westergren, T. Niemi, M. Taittonen, J. Laine, N.J. Savisto, S. Enerback, P. Nuutila, Functional brown adipose tissue in healthy adults, N Engl J Med. 360 (2009) 1518-1525.
[14] H.L. Garstka, W.E. Schmitt, J. Schultz, B. Sogl, B. Silakowski, A. Perez-Martos, J. Montoya, R.J. Wiesner, Import of mitochondrial transcription factor A (TFAM) into rat liver mitochondria stimulates transcription of mitochondrial DNA, Nucleic Acids Res. 31 (2003) 5039-5047.
[15] Z. Wu, P. Puigserver, B. M. Spiegelman, Transcriptional activation of adipogenesis, Curr Opin Cell Biol. 11 (1999) 689-694.
[17] L.A. Foellmi-Adams, B.M. Wyse, D. Herron, J. Nedergaard, R.F. Kletzien, Induction of uncoupling protein in brown adipose tissue. Synergy between norepinephrine and pioglitazone, an insulin-sensitizing agent, Biochem Pharmacol. 52 (1996) 693-701.
[18] M. Mensink, M.K. Hesselink, A.P. Russell, G. Schaart, J.P. SeIs, P. Schrauwen, Improved skeletal muscle oxidative enzyme activity and restoration of PGC-I alpha and PPAR beta/ delta gene expression upon rosiglitazone treatment in obese patients with type 2 diabetes mellitus, Int J Obes (Lond). 31 (2007) 1302-1310.
[19] L. Lehr, K. Canola, C. Asensio, M. Jimenez, F. Kuehne, J.P. Giacobino, P. Muzzin, The control of UCP1 is dissociated from that of PGC-I alpha or of mitochondriogenesis as revealed by a study using beta-less mouse brown adipocytes in culture, FEBS Lett. 580 (2006) 4661-4666.
[20] O. Champigny, B. R. Holloway, D. Ricquier, Regulation of UCP gene expression in brown adipocytes differentiated in primary culture. Effects of a new beta-adrenoceptor agonist, MoI Cell Endocrinol. 86 (1992) 73-82.
[24] M. Jimenez, C. Yvon, L. Lehr, B. Leger, P. Keller, A. Russell, F. Kuhne, P. Flandin, J. P. Giacobino, P. Muzzin, Expression of uncoupling protein-3 in subsarcolemmal and intermyofibrillar mitochondria of various mouse muscle types and its modulation by fasting, Eur J Biochem. 269 (2002) 2878-2884.
[26] Tseng YH, Kokkotou E, Schulz TJ, Huang TL, Winnay JN, Taniguchi CM, Tran TT, Suzuki R, Espinoza DO, Yamamoto Y, Ahrens MJ, Dudley AT, Norris AW, Kulkarni RN, Kahn CR. New role of bone morphogenetic protein 7 in brown adipogenesis and energy expenditure. Nature. 2008 Aug 21;454(7207):1000-4. PubMed PMID: 18719589; PubMed Central PMCID: PMC2745972.
[27] Zhang H, Schulz TJ, Espinoza DO, Huang TL, Emanuelli B, Kristiansen K, Tseng YH. Cross talk between insulin and bone morphogenetic protein signaling systems in brown adipogenesis. Mol Cell Biol. 2010 Sep;30(17):4224-33. Epub 2010 Jun 28. PubMed PMID: 20584981; PubMed Central PMCID: PMC2937545.
[28] Schulz TJ, Huang TL, Tran TT, Zhang H, Townsend KL, Shadrach JL, Cerletti M, McDougall LE, Giorgadze N, Tchkonia T, Schrier D, Falb D, Kirkland JL, Wagers AJ, Tseng YH. Identification of inducible brown adipocyte progenitors residing in skeletal muscle and white fat. Proc Natl Acad Sci U S A. 2011 Jan 4;108(1):143-8. Epub 2010 Dec 20. PubMed PMID: 21173238; PubMed Central PMCID: PMC3017184.
[29] Farmer SR, Boss O (2012) Recruitment of Brown Adipose Tissue as a Therapy for Obesity-Associated Diseases. Frontiers in Endocrinology 3.
[30] Zhang N, Chen W, Zhou X, Xie X, Meng A, et al. (2013) C333H ameliorated insulin resistance through selectively modulating PPARgamma in brown adipose tissue of db/db mice. Biol Pharm Bull.
[31] Yoneshiro T, Aita S, Matsushita M, Kayahara T, Kameya T, et al. (2013) Recruited brown adipose tissue as an antiobesity agent in humans. J Clin Invest.
[32] Vijgen GH, Sparks LM, Bouvy ND, Schaart G, Hoeks J, et al. (2013) Increased Oxygen Consumption in Human Adipose Tissue From the "Brown Adipose Tissue" Region. J Clin Endocrinol Metab.
[33] Vijgen GH, van Marken Lichtenbelt WD (2013) Brown adipose tissue: clinical impact of a rediscovered thermogenic organ. Front Biosci (Elite Ed) E5: 823-833.
[34] van der Lans AA, Hoeks J, Brans B, Vijgen GH, Visser MG, et al. (2013) Cold acclimation recruits human brown fat and increases nonshivering thermogenesis. The Journal of Clinical Investigation 123: 3395-3403.
[35] Stanford KI, Middelbeek RJ, Townsend KL, An D, Nygaard EB, et al. (2013) Brown adipose tissue regulates glucose homeostasis and insulin sensitivity. J Clin Invest 123: 215-223.
[36] Pasanisi F, Pace L, Fonti R, Marra M, Sgambati D, et al. (2013) Evidence of Brown Fat Activity in Constitutional Leanness. J Clin Endocrinol Metab.
[37] Yin H, Pasut A, Soleimani VD, Bentzinger CF, Antoun G, et al. (2013) MicroRNA-133 Controls Brown Adipose Determination in Skeletal Muscle Satellite Cells by Targeting Prdm16. Cell Metab 17: 210-224.

## Claims

1. An ex vivo method of promoting brown adipogenesis using an agent being Pituitary adenylate cyclase-activating polypeptide (PACAP) propeptide of 55 aa (aa 25-79).

2. The method of claim 1, said method comprising contacting a cell with the agent.

3. The method of claim 2, wherein the cell is a brown adipose tissue (BAT) progenitor cell isolated from human skeletal muscle.

4. The method of claim 3, wherein the cell is positive for CD34, and/or wherein the cell is negative for CD31.

## Patentansprüche

1. Ein ex vivo Verfahren zur Förderung der braunen Adipogenese unter Verwendung eines Mittels, das ein Hypophysenadenylatzyklase-aktivierendes Polypeptid (PACAP) Propeptid aus 55 AA (AA 25-79) ist.

2. Das Verfahren nach Anspruch 1, wobei das Verfahren das Kontaktieren einer Zelle mit dem Mittel umfasst.

3. Das Verfahren nach Anspruch 2, wobei die Zelle eine Vorläuferzelle des braunen Fettgewebes (BAT) ist, die aus humanem Skelettmuskel isoliert ist.

4. Das Verfahren nach Anspruch 3, wobei die Zelle positiv für CD34 ist und/oder wobei die Zelle negativ für CD31 ist.

## Revendications

1. Méthode ex vivo de promotion d'une adipogenèse brune utilisant un agent qui est un propeptide de polypeptide activant l'adénylate cyclase pituitaire (PACAP) de 55 aa (aa 25 à 79).

2. Méthode selon la revendication 1, ladite méthode comprenant la mise en contact d'une cellule avec l'agent.

3. Méthode selon la revendication 2, dans laquelle la cellule est une cellule progénitrice de tissu adipeux brun (BAT) isolée à partir de muscle squelettique humain.

4. Méthode selon la revendication 3, dans laquelle la cellule est positive pour CD34, et/ou dans laquelle la cellule est négative pour CD31.
